(19) Europäisches Patentamt
European Patent Office
Office européen des brevets

(11) Numéro de publication: **0 374 445 A1**

(12) # DEMANDE DE BREVET EUROPEEN

(21) Numéro de dépôt: **89120338.2**

(22) Date de dépôt: **03.11.89**

(51) Int. Cl.5: **C07C 49/753, C07C 49/517, C07C 43/196, C07D 317/44, C11B 9/00, A61K 7/46, C07C 45/29, C07C 45/30, C07C 41/28**

(30) Priorité: **23.12.88 CH 4791/88**

(43) Date de publication de la demande: **27.06.90 Bulletin 90/26**

(84) Etats contractants désignés: **CH DE FR GB LI NL**

(71) Demandeur: **FIRMENICH SA**
**Case Postale 239**
**CH-1211 Genève 8(CH)**

(72) Inventeur: **Giersch, Wolfgang Klaus**
**323, rue de Bernex**
**CH-1233 Bernex(CH)**
Inventeur: **Schulte-Elte, Karl-Heinrich**
**44, chemin de Carabot**
**CH-1213 Onex(CH)**

(74) Mandataire: **Salvadori, Giuseppe, Dr.**
**c/o Firmenich S.A. Case Postale 239**
**CH-1211 Genève 8(CH)**

(54) **Nouveaux composés cycliques oxygénés, leur utilisation à titre d'ingrédients parfumants, procédé pour leur préparation.**

(57) Les composés de formule

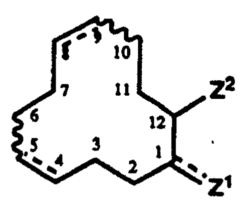

(I)

dans laquelle

a) les pointillés désignent l'emplacement d'une liaison simple ou double en positions 4 et 8 et d'une liaison carbone-oxygène simple en position 1, les lignes ondulées définissent une liaison C-C de configuration E ou Z lorsque la liaison en positions 4 et 8 est double, et les symboles $Z^1$ et $Z^2$, pris conjointement, définissent un groupe acétal ou cétal cyclique de formule

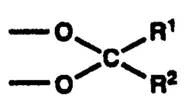

dans laquelle les symboles $R^1$ et $R^2$, identiques ou différents, représentent chacun un radical alkyle, de chaîne linéaire ou ramifiée, contenant de 1 à 6 atomes de carbone, ou l'un de ces symboles représente un atome d'hydrogène et l'autre représente un radical alkyle défini comme ci-dessus ;

ou dans laquelle

b) le symbole $Z^1$ représente un atome d'oxygène ou un groupe OH, les pointillés représentent

EP 0 374 445 A1

l'emplacement d'une liaison simple ou double en positions 4 et 8, et d'une liaison carbone-oxygène simple ($Z^1$ = OH) ou double ($Z^1$ = O) en position 1, les lignes ondulées représentent une liaison C-C de configuration E ou Z lorsque la liaison en positions 4 et 8 est double, et le symbole $Z^2$ représente un groupe $OR^3$, où $R^3$ est un reste alkyle, de chaîne linéaire ou ramifiée, contenant de 1 à 6 atomes de carbone, sont utiles à titre d'ingrédients parfumants. Ils servent à conférer des notes de type épicé-aldéhydé à des parfums et produits parfumés. Les 12-pentyloxy-4,8-cyclododécadién-1-one, 12-isobutoxy-4,8-cyclododécadién-1-one et 12-(2,2-dimethylpropoxy)-4,8-cyclopentadécadién-1-one sont cités à titre de composés préférentiels.

On décrit également un procédé de préparation des composés de formule (I).

# Nouveaux composés cycliques oxygénés, leur utilisation à titre d'ingrédients parfumants, procédé pour leur préparation

L'invention a trait au domaine de la parfumerie. Plus particulièrement, elle a pour objet l'utilisation à titre d'ingrédient parfumant d'un composé de formule

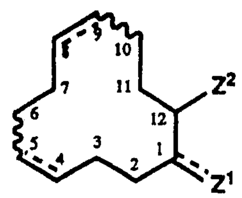

(I)

dans laquelle

a) les pointillés désignent l'emplacement d'une liaison simple ou double en positions 4 et 8 et d'une liaison carbone-oxygène simple en position 1, les lignes ondulées définissent une liaison C-C de configuration E ou Z lorsque la liaison en positions 4 et 8 est double, et les symboles $Z^1$ et $Z^2$, pris conjointement, définissent un groupe acétal ou cétal cyclique de formule

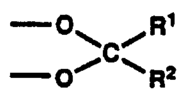

dans laquelle les symboles $R^1$ et $R^2$, identiques ou différents, représentent chacun un radical alkyle, de chaîne linéaire ou ramifiée, contenant de 1 à 6 atomes de carbone, ou l'un de ces symboles représente un atome d'hydrogène et l'autre représente un radical alkyle défini comme ci-dessus ;

ou dans laquelle

b) le symbole $Z^1$ représente un atome d'oxygène ou un groupe OH, les pointillés représentent l'emplacement d'une liaison simple ou double en positions 4 et 8, et d'une liaison carbone-oxygène simple ($Z^1$ = OH) ou double ($Z^1$ = O) en position 1, les lignes ondulées représentent une liaison C-C de configuration E ou Z lorsque la liaison en positions 4 et 8 est double, et le symbole $Z^2$ représente un groupe $OR^3$, où $R^3$ est un reste alkyle, de chaîne linéaire ou ramifiée, contenant de 1 à 6 atomes de carbone.

Elle a également pour objet une composition parfumante et un produit parfumé contenant un composé de formule (I) à titre d'ingrédient parfumant. Elle a, en outre, pour objet un procédé pour la préparation d'un composé de formule (I) définie ci-dessus, caractérisé en ce que :

a) on fait réagir, en présence d'un agent acide, un diol de formule

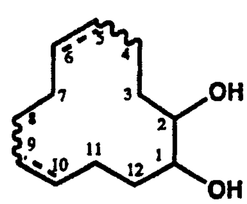

(II)

dans laquelle les pointillés désignent l'emplacement d'une liaison simple ou double en positions 5 et 9, et les lignes ondulées servent à définir une liaison C-C de configuration E ou Z lorsque la liaison en positions 5 et 9 est double, avec un composé de formule

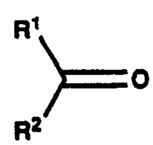

dans laquelle $R^1$ et $R^2$ sont définis comme à la revendication 1, pour obtenir un cétal de formule

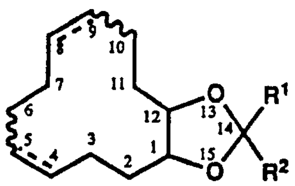

(III)

dans laquelle les lignes ondulées et les symboles $R^1$ et $R^2$ sont définis ci-dessus, et les pointillés désignent l'emplacement d'une liaison simple ou double en positions 4 et 8 ;

b) on fait réagir le cétal de formule (III) avec un agent réducteur pour obtenir un hydroxy-éther de formule

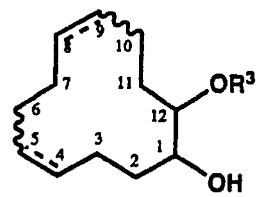

(IV)

dans laquelle les pointillés désignent l'emplacement d'une liaison simple ou double en positions 4 et 8, les lignes ondulées représentent une liaison C-C de configuration E ou Z lorsque la liaison en positions 4 et 8 est double, et le symbole $R^3$ est défini comme à la revendication 1 ;

c) on fait réagir l'hydroxy-éther de formule (IV) avec un agent oxydant pour obtenir un céto-éther de formule

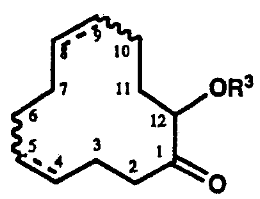

(V)

dans laquelle les pointillés, les lignes ondulées et le symbole $R^3$ sont définis comme en b).

L'invention concerne également les composés nouveaux de formule (I) définie ci-dessus, étant entendu que lorsque $Z^1$ est l'oxygène, $R^3$ ne peut pas être $CH_3$.

En effet, parmi les composés de formule (I), les 2-méthoxycyclododécanone et 12-méthoxy-4,8-cyclododécadién-1-one ont été décrits dans la littérature scientifique, mais sans que pour autant des propriétés odoriférantes leur aient été reconnues [voir à ce sujet L. Chow-Yan, S. K. Pillay, H. Richard, Can. J. Chem., (1979), 57, 2923 et B. Foehlisch, W. Gottstein, R. Kaiser, I. Wanner, Tetrahedron Lett. (1980), 21, 3005].

Nous avons découvert que ces composés connus ainsi que les composés nouveaux de formule (I) définis ci-dessus possédaient des propriétés odorantes fort intéressantes et, de ce fait, pouvaient être employés avantageusement pour le parfumage de produits divers, tels les savons, les produits cosmétiques, les shampoings, les détergents ou les produits d'entretien, ainsi que pour la manufacture de parfums, bases parfumantes ou concentrés. Ces composés conviennent également pour le parfumage d'adoucissants et de revitalisants textiles.

A titre de composés (I) préférentiels, on peut citer les composés de formule (I) dans laquelle le symbole $Z^1$ est l'oxygène, les pointillés désignent l'emplacement d'une liaison double en positions 4 et 8, et d'une liaison carbone-oxygène double en position 1, les lignes ondulées définissent une liaison C-C de configuration E ou Z, et le symbole $Z^2$ représente un groupe $OR^3$, où $R^3$ est un radical alkyle, de chaîne linéaire ou ramifiée, contenant de 2 à 6 atomes de carbone.

Plus particulièrement, les composés cités ci-dessus à titre préférentiel, à savoir les dérivés céto-éthers du 4,8-cyclododécadiène, présentent des propriétés odorantes surprenantes. En effet, contrairement à ce que l'on pourrait prévoir sur la base d'études publiées sur d'autres dérivés du cyclododécane [voir à ce

sujet H. Aebi, E. Baumgartner, H. P. Fiedler et G. Ohloff in Kosmetika, Riechstoffe und Lebensmittelzusatzstoffe, ed. Georg Thieme Verlag, Stuttgart (1978)] et en dépit de leur structure de céto-éthers, ces composés développent des odeurs de type aldéhydé.

Parmi ces dérivés céto-éthers, on mentionnera à titre préférentiel le 12-pentyloxy-4,8-cyclododécadién-1-one. Ce corps est particulièrement intéressant car il développe une odeur fruitée-calamus, aromatique dans la direction du coriandre, avec une forte note aldéhydée. Par ailleurs, il développe une tonalité épicée dans la direction de la cascarille. La combinaison de ces deux côtés, d'une part fruité-calamus, d'autre part épicé-cascarille, est unique pour un corps synthétique et sa structure de céto-éther confère à ce composé une stabilité supérieure à celle des aldéhydes. Ses multiples facettes lui confèrent un large éventail d'utilisations et ce composé convient particulièrement bien à la préparation de compositions parfumantes de type varié, auxquelles il donne plus de volume et de puissance, en plus des tonalités qui le caractérisent. Son utilisation dans des eaux de toilette de type masculin, par exemple, ou dans des lotions après rasage, se révèle particulièrement heureuse. En outre, il peut être utilisé dans des produits parfumés, notamment dans des détergents en poudre, le parfum prenant alors plus de volume.

On mentionnera encore à titre de composés préférentiels selon l'invention, les 12-(2,2-diméthylpropoxy)-4,8-cyclododécadién-1-one et 12-isobutoxy-4,8-cyclododécadién-1-one qui peuvent être employés pour des applications semblables à celles décrites ci-dessus.

Comme précité, les composés selon l'invention, et, notamment, les dérivés céto-éthers du cyclododécane et du cyclododécadiène peuvent être utilisés dans des compositions parfumantes, bases et concentrés, ainsi que dans la préparation de produits parfumés tels que savons, détergents, adoucissants, produits d'entretien et dans des préparations cosmétiques.

Les quantités de ces composés à utiliser pour obtenir un effet parfumant, notamment dans des compositions parfumantes, varient entre 5 et 10% (poids) environ, mais ces chiffres sont présentés uniquement à titre indicatif, étant entendu que ces quantités peuvent varier de façon considérable en fonction des ingrédients de base auxquels ces composés sont ajoutés.

Selon l'invention, les produits de départ pour la préparation des composés de formule (I) sont soit le 1,2-cyclododécadiénol, soit le 5,9-cyclododécadiène-1,2-diol, représentés par la formule générale

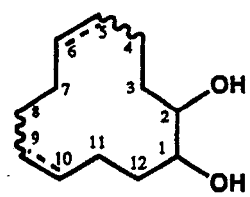

(II)

dans laquelle les pointillés désignent l'emplacement d'une liaison simple ou double en positions 5 et 9, et les lignes ondulées servent à définir une liaison C-C de configuration E ou Z lorsque la liaison en positions 5 et 9 est double. En faisant réagir ces diols avec un composé de formule

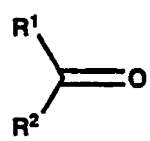

dans laquelle les symboles $R^1$ et $R^2$ sont définis comme précédemment, on obtient des cétals de formule

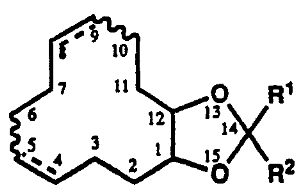

(III)

dans laquelle les lignes ondulées et les symboles $R^1$ et $R^2$ sont définis ci-dessus, et les pointillés désignent l'emplacement d'une liaison simple ou double en positions 4 et 8.

Selon l'invention, ladite réaction s'effectue en présence d'un agent acide qui peut être un acide

protonique minéral ou organique, ou encore un acide de type Lewis.

La réaction s'effectue, en outre, en présence d'un solvant organique tel que l'éther de pétrole. Les diols de formule (II) utilisés à titre de produits de départ selon le procédé de l'invention, peuvent être obtenus soit sur le marché, soit par des procédés de préparation connus [voir M. Ohno, M. Okamoto, S. Torimitsu, Bull. Chem. Soc. Japan (1966), 39, 316, L. I. Zakharkin, V. V. Korneva, Dokl. Akad. Nauk. SSSR (1960), 132, 1078 et V. Kosswig, Chem. Zeitg. (1972), 96, 373].

Tels que directement issus de la synthèse, les composés de formule (III) dans laquelle les pointillés représentent des liaisons doubles peuvent se présenter sous forme de mélanges de trois formes isomériques qui résultent de l'isomérie de configuration E ou Z des liaisons C-C en positions 5 et 9. Pour des raisons d'ordre pratique et économique, on utilisera de préférence des mélanges d'isomères tels que provenant directement de la synthèse, en tant que produits de départ pour les synthèses des composés de formule (IV), décrite ci-après.

Selon l'invention, par réaction des cétals de formule (III) avec un agent réducteur, on obtient les hydroxy-éthers de formule

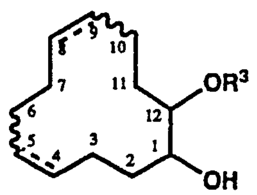

(IV)

dans laquelle les pointillés désignent l'emplacement d'une liaison simple ou double en positions 4 et 8, les lignes ondulées représentent une liaison C-C de configuration E ou Z lorsque la liaison en positions 4 et 8 est double, et le symbole $R^3$ est défini comme à la revendication 1.

La réduction du composé cétal s'effectue dans des conditions connues au moyen, par exemple, de $LiAlH_4/AlCl_3$ [voir E. L. Eliel, V. G. Badding, M. N. Rerick, J. Am. Chem. Soc. (1962), 84, 2371] ou de DIBAH (diisobutylaluminium hydrure) [voir K. Ishikara, A. Mori, H. Yamamoto, Tetrahedron Lett., (1987), 6613].

Les composés de formule (IV) issus directement de cette synthèse peuvent se présenter, lorsqu'il s'agit d'hydroxy-éthers dérivés du cyclododécane, sous forme de mélanges de deux formes isomériques qui correspondent à la configuration E ou Z des liaisons C-O en positions 1 et 12. Lorsque les composés de formula (IV) sont des hydroxy-éthers dérivés du 4,8-cyclododécadiène, ils peuvent se présenter sous plusieurs formes isomériques, résultant de la combinaison de l'isomérie E/Z des liaisons C-O en positions 1 et 12, avec l'isomérie E/Z des liaisons C-C déjà citée. Encore une fois, on préfère utiliser des mélanges d'isomères tels que provenant directement de la synthèse pour les applications en parfumerie, ainsi que comme produits de départ pour la synthèse des céto-éthers de formule (V) décrits ci-après.

Selon le procédé de l'invention, on prépare des céto-éthers de formule (V)

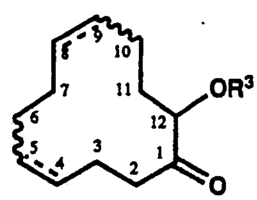

(V)

dans laquelle les pointillés représentent une liaison simple ou double en positions 4 et 8, les lignes ondulées représentent une liaison C-C de configuration E ou Z lorsque la liaison en positions 4 et 8 est double, et le symbole $R^3$ sert à définir un radical alkyle, de chaîne linéaire ou ramifiée, contenant de 1 à 6 atomes de carbone, en faisant réagir des hydroxy-éthers de formule (IV) avec un agent oxydant. La réaction d'oxydation de l'hydroxy-éther s'effectue dans des conditions connues, en utilisant comme agent oxydant, par exemple, le PDC (pyridinium dichromate) [voir E. J. Corey, G. Schmidt, Tetrahedron Lett. (1979), 399] ou le PCC (pyridinium chlorochromate) [voir E. J. Corey, J. W. Suggs, Tetrahedron Lett. (1975), 2647].

Les céto-éthers de formule (V), dans laquelle les pointillés désignent une liaison double, directement issus de la synthèse, peuvent prendre trois formes isomériques, résultant de la configuration E ou Z des liaisons C-C adjacentes à la liaison double. Les mélanges d'isomères tels que provenant de la synthèse sont préférentiellement utilisés en tant que produits parfumants, pour les raisons déjà invoquées.

Les tableaux I à III présentent des composés préparés selon le procédé de l'invention, ainsi que leurs points d'ébullition et le rendement de la synthèse correspondante. Le tableau I donne également, en regard de chacun des composés obtenus, l'aldéhyde ou la cétone utilisé(e) comme produit de départ pour sa préparation.

TABLEAU I - Cétals de formule générale (III)

| Composé | Composé de départ de formule R1 R2 C=O | Point d'ébullition (four à boules) °C/Pa | Rendement % |
|---|---|---|---|
| 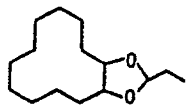 | 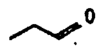 | 120/4,0 | 63 |
| 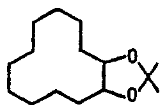 | 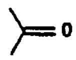 | 100/3,3 | 89 |
| 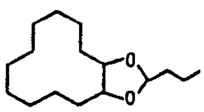 | 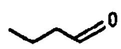 | 120/4,0 | 95 |
| 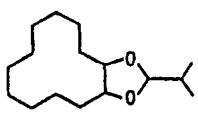 | 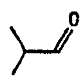 | 130/5,4 | 90 |
| 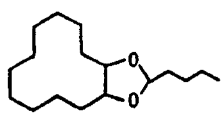 | 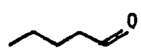 | 125/4,4 | 92 |
| 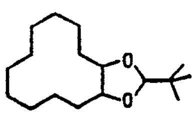 | 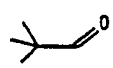 | 115/6,0 | 76 |
| 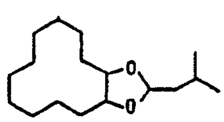 | 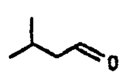 | 120/4,0 | 93 |
| 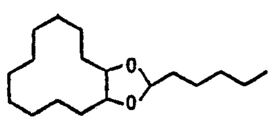 | 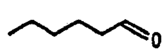 | 125/4,0 | 92 |
| 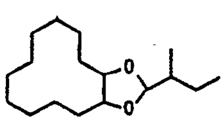 | 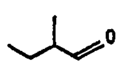 | 105/3,7 | 32 |

<u>TABLEAU I</u> - Cétals de formule générale (III)
(suite)

| Composé | Composé de départ de formule 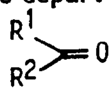 | Point d'ébullition (four à boules) °C/Pa | Rendement % |
|---|---|---|---|
| 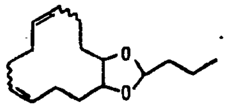 | 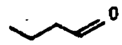 | 135/5,4 | 80 |
| 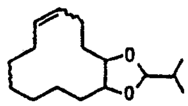 | 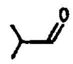 | 100/4,0 | 93 |
| 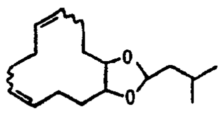 | 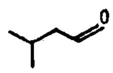 | 120/3,7 | 92 |
| 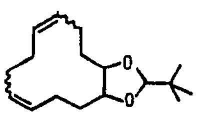 | 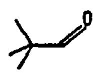 | 115/6,0 | 78 |
| 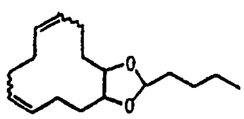 | 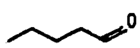 | 130/6,0 | 90 |
| 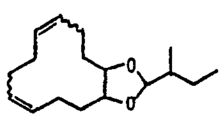 | 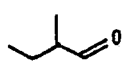 | 100/4,0 | 93 |
| 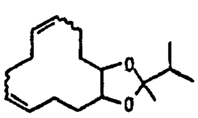 | 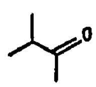 | 105/6,1 | 42 |
| 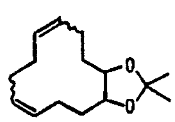 | 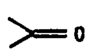 | 95/3,1 | 93 |
| 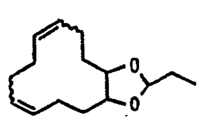 | 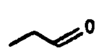 | 95/4,0 | 73 |

TABLEAU II - Composés de formule générale (IV)

| | 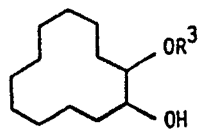 | | 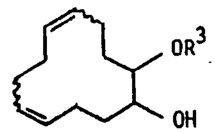 | |
|---|---|---|---|---|
| $R^3$ | Point d'ébullition (four à boules) °C/Pa | Rend. % | Point d'ébullition (four à boules) °C/Pa | Rend. % |
| $-CH_2CH_2CH_3$ | 130/5,0 | 82 | 130/5,4 | 81 |
| 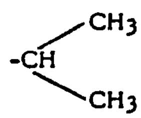 | 120/5,2 | 90 | 110/3,5 | 89 |
| $-CH_2CH_2CH_2CH_3$ | 130/3,5 | 89 | 125/3,7 | 88 |
| 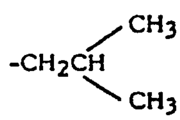 | 110/3,7 | 85 | 100/4,6 | 79 |
| $-CH_2CH_2CH_2CH_2CH_3$ | 150/4,2 | 93 | 120/7,2 | 88 |
| 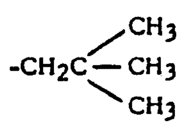 | 130/4,0 | 83 | 115/4,0 | 92 |
| 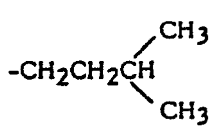 | 120/4,2 | 81 | 120/3,9 | 86 |
| $-CH_2CH_2CH_2CH_2CH_2CH_3$ | 150/4,2 | 97 | - | - |
| 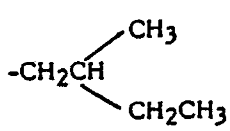 | 110/3,0 | 78 | 110/4,0 | 72 |
| 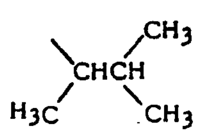 | - | - | 145/6,8 | 73 |

TABLEAU III - Composés de formule générale (V)

| $R^3$ | 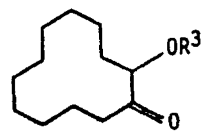 Point d'ébullition (four à boules) °C/Pa | Rend. % | 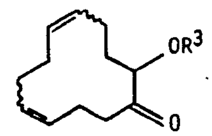 Point d'ébullition (four à boules) °C/Pa | Rend. % |
|---|---|---|---|---|
| $-CH_2CH_2CH_3$ | 95/9,0 | 83 | 105/3,8 | 81 |
| 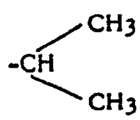 | 115/5,6 | 86 | 95/5,2 | 77 |
| $-CH_2CH_2CH_2CH_3$ | 115/4,2 | 83 | 95/5,4 | 51 |
| 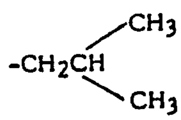 | 100/4,6 | 69 | 105/5,4 | 73 |
| $-CH_2CH_2CH_2CH_2CH_3$ | 130/4,0 | 26 | 120/7,2 | 75 |
| 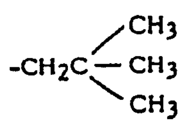 | 95/3,5 | 89 | 95/4,0 | 86 |
| 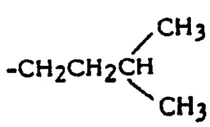 | 95/3,5 | 68 | 100/4,6 | 73 |
| $-CH_2CH_2CH_2CH_2CH_2CH_3$ | 160/7,2 | 45 | - | - |
| 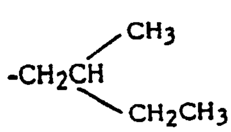 | 115/4,8 | 68 | 105/4,2 | 60 |
| 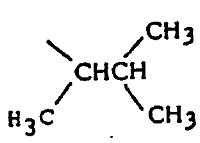 | - | - | 140/5,8 | 81 |

L'invention sera illustrée, de façon non limitative, par les exemples ci-après qui ont trait à des composés préférés de l'invention.

Exemple 1

a) Dans un ballon à deux cols de 250 ml muni d'un séparateur d'eau ont été chargés 5 g de 5,9-cyclododécadiène-1,2-diol, 2,4 g d'aldéhyde valérianique, 1 spatule d'acide p-toluènesulfonique et 200 ml d'éther de pétrole 80/100, et le mélange a été porté à reflux pendant 1 h. Le mélange de la réaction a ensuite été versé sur glace, neutralisé avec de la saumure, séché et concentré. Après distillation au four à boules, on a obtenu 6,06 g de 14-butyl-13,15-dioxa-bicyclo[10.3.0] pentadéca-4,8-diène.
Eb. 130° C/6,0 Pa ; rend. 90%
IR : pas de fonction
RMN (60 MHz) : 0,9(tr,J = 6Hz,3H) ; 3,6-4,4(m,2H) ; 4,8-5,15(m,1H) ; 5,3-5,7(m,4H) delta ppm ;
CG-SM : (2 pics, environ 1:1) :
1) 264 $M^+$,3), 207(21), 161(83), 133(27), 119(38), 91(48), 79(91), 67(100), 41(81) ;
2) 264 ($M^+$, inférieur à 1), 207(31), 161(100), 133(29), 119(41), 91(48), 79(92), 67(88), 41(66).

b) 38,2 ml de DIBAH 1,2 M dans le toluène ont été chargés dans un ballon de 100 ml à trois cols et une solution de toluène du cétal préparé en a) obtenue à partir de 6,15 g de 14-butyl-13,15-dioxabicyclo-[10.3.0] pentadéca-4,8-diène et 20 ml de toluène, a été ajoutée goutte à goutte. Après 1 h à reflux, le mélange de la réaction a ensuite été versé sur glace et acidifié pour dissoudre les sels d'aluminium, et par la suite neutralisé, séché et concentré. Après distillation dans un four à boules, on a obtenu 5,76 g de 1 2-pentyloxy-4,8-cyclododéca-dién-1-ol.
Eb. 130° C/4,2 Pa ; rend. 93%
IR : 3430 $cm^{-1}$
RMN (60 MHz) : 0,91(tr,J = 6Hz,3H) ; 2,4(OH) ; 3,42(m,2H) ; 3,8(m,2H) ; 5,2-5,7(m,4H) delta ppm ;
CG-SM (1 pic large) : 266 ($M^+$,19), 196(3), 178(6), 133(7), 97(16), 81(36), 67(28), 55(30), 43(100).

c) Dans un ballon de 250 ml ont été chargés 8,8 g de terre d'infusoires et une solution de 5,39 g de 12-pentyloxy-4,8-dodécadién-1-ol dans 50 ml de dichlorométhane. 8.8 g de PCC ont ensuite été ajoutés par portions et le tout agité pendant 2 h à température ambiante. On a ajouté de l'éther éthylique au mélange de la réaction pour précipiter les sels de chrome et, ensuite, filtré sur $SiO_2$, concentré et distillé au four à boules. 4 g de 12-pentyloxy-4,8-cyclododécadién-1-one furent obtenus.
Eb. 120° C/7,2 Pa ; rend. 75%
IR : 1715 $cm^{-1}$
RMN (60 MHz) : 0,91(tr,J = 6Hz,3H) ; 3,25-3,85(m,3H) ; 5,0-5,7(m,4H) delta ppm ;
CG-SM : (2 pics, environ 1:1) :
1) 264 ($M^+$,0), 196(2), 176(1), 160(3), 133(5), 119(11), 107(8), 94(12), 79(28), 67(33), 55(31), 43(100)
2) 264 ($M^+$,0), 196(2), 133(5), 119(8), 97(13), 79(33), 67(32), 55(31), 43(100).
Qualification olfactive : épicé-aldéhydé, légère note métallique, puissant.

Exemple 2

a) 5 g de 5,9-cyclododécadién-1,2-diol, 2,4 g d'aldéhyde pivalique, 1 spatule d'acide p-toluènesulfonique et 100 ml d'éther de pétrole 80/100 ont été chargés dans un ballon à deux cols de 250 ml muni d'un séparateur d'eau et portés à reflux pendant 2 h. Le mélange de la réaction fut ensuite versé sur glace, lavé à neutralité avec de la saumure, séché et concentré. Après distillation au four à boules, on a obtenu 5,2 g de 14-tert-butyl-13,15-dioxabicyclo[10.3.0]pentadéca-4,8-diène.
Eb. 115° C/6,0 Pa ; rend. 78%
IR : pas de fonction
RMN (60 MHz) ; 0,9 et 0,92(2s,9H) ; 3,7-4,4(m,2H) ; 4,5 et 4,62 (2s,1H) ; 5,3-5,7(m,4H) delta ppm ;
CG-SM : (2 pics, environ 1:2) :
1) 264 ($M^+$,0), 263(1), 207(57), 161(100), 133(27), 119(26), 91(37), 79(48), 67(50), 41(78)
2) 264($M^+$,1), 207(57), 161(100), 133(28), 119(29), 91(31), 79(50), 67(49), 41(73).

b) Procédé de préparation analogue à celui décrit dans l'Exemple 1 b), sauf que 5,2 g de 14-tert-butyl-13,15-dioxabicyclo[10.3.0]pentadéca-4,8-diène et 32,8 ml de DIBAH 1,2 M dans le toluène ont été

utilisés. Le mélange fut repris dans de l'éther après l'acidification, lavé avec saumure, séché, concentré et distillé au four à boules. On a obtenu 4,82 g de 12-(2,2-diméthyl-propoxy)-4,8-cyclododécadién-1-ol.
Eb. 115° C/4,0 Pa ; rend. 92%
IR : 3420 $cm^{-1}$
RMN (60 MHz) : 0,91(s,9H) ; 3,12(s,2H) ; 3,4(m,1H) ; 3,85(m,1H) ; 5,3-5,6(m,4H) delta ppm ;
CG-SM : (2 pics, environ 9:1) :
1) 266 ($M^+$,1), 196(9), 133(8), 109(7), 93(13), 81(32), 71(85), 43(100)
2) 266 ($M^+$,0), 252(4), 196(2), 178(3), 133(10), 119(12), 109(14), 97(31), 81(80), 67(66), 57(78), 41-(100).

c) Procédé analogue à celui décrit dans l'Exemple 1 c), en utilisant comme produits de départ 4,82 g de 12-(2,2-diméthylpropoxy)-4,8-dodécadién-1-ol, 7,8 g de PCC et 50 ml de dichlorométhane. L'agitation a été effectuée pendant 1 h. On a obtenu 4,11 g de 12-(2,2-diméthyl-propoxy)-4,8-cyclododécadién-1-one.
Eb. 95° C/4,0 Pa ; rend. 86%
IR : 1710 $cm^{-1}$
RMN (60 MHz) : 0,92 et 0,96 (2s,environ 2:1,9H) ; 3,02(s,2H) ; 3,7(m,1H) ; 5,2-5,6(m,4H) delta ppm ;
CG-SM : (2 pics, environ 2:1) :
1) 264 ($M^+$,15), 207(1), 196(7), 176(3), 148(4), 133(4), 120(5), 109(4), 93(7), 79(10), 71(81), 43(100)
2) 264 ($M^+$,17), 207(2), 196(7), 176(3), 166(5), 149(3), 133(3), 119(5), 97(9), 79(9), 71(88), 43(100).
Qualification olfactive : épicé-vert-marine, odeur naturelle calamus-cardamome.

## Exemple 3

a) On a procédé comme dans l'Exemple 1 a), sauf que 5 g de 5,9-cyclododécadién-1,2-diol, 2,02 g d'aldéhyde isobutyrique et 100 ml d'éther de pétrole 80/100 ont été utilisés. On a obtenu 5,92 g de 14-isopropyl-13,15-dioxa-bicyclo[10.3.0] pentadéca-4,8-diène.
Eb. 100° C/4,0 Pa ; rend. 93%
IR : pas de fonction
RMN (60 MHz) : 0,93(d,J=7Hz,6H) ; 3,7-4,4(m,2H) ; 4,6 et 4,76 (2d, J environ=5Hz,1H) ; 5,3-5,7(m,4H) delta ppm ;
CG-SM (1 pic large) : 250 ($M^+$,2), 107(52), 161(100), 133(27), 119(32), 91(36), 79(56), 67(52), 41(73).

b) On a procédé comme dans l'Exemple 2 b), sauf que 5,92 g de 14-isopropyl-13,15-dioxabicyclo-[10.3.0]pentadéca-4,8-diène et 39,5 ml de DIBAH 1,2 M dans le toluène ont été utilisés. On a obtenu 4,71 g de 12-isobutoxy-4,8-cyclododécadién-1-ol.
Eb. 100° C/4,6 Pa ; rend. 79%
IR : 3450 $cm^{-1}$
RMN (60MHz) : 0,92(d,J=7Hz,6H) ; 2,2(OH) ; 3,32(d,J=7Hz,2H) ; 3,2-4,2(m,2H) ; 5,2- 5,6(m,4H) delta ppm ;
CG-SM (1 pic) : 252($M^+$,12), 196(12), 178(7), 133(9), 109(9), 81(29), 67(31), 57(75), 41(100).

c) 4,71 g de 12-isobutoxy-4,8-cyclododécadién-1-ol ont été solubilisés dans 50 ml de dichlorométhane et 8 g de terre d'infusoires ajoutés en suspension à cette solution. Après agitation, on a additionné 8,05 g de PCC par portions, l'agitation ayant été maintenue pendant 2 h à température ambiante. Les sels de chrome ont ensuite été précipités avec de l'éther éthylique et le mélange a été filtré sur $SiO_2$ et concentré. Après distillation au four à boules, on a obtenu 3,43 g de 12-isobutoxy-4,8-cyclododécadién-1-one.
Eb. 105° C/5,4 Pa ; rend. 73%
IR : 1715 $cm^{-1}$
RMN (60MHz) : 0,91(d,J=7Hz,6H) ; 3,15(d,J=7Hz,2H) ; 3,7(m,1H) ; 5,1-5,6(m,4H) delta ppm ;
CG-SM (2 pics, environ 3:1) :
1) 250($M^+$,28), 182(9), 133(7), 119(11), 97(12), 79(16), 67(24), 57(89), 41(100)
2) 250($M^+$,29), 182(12), 166(7), 120(9), 107(9), 97(12), 79(27), 67(27), 57(100), 41(96)
Qualification olfactive : épicé, aldéhydé, côté cardamome/calamus.

## Exemple 4

On a préparé une composition parfumante de base pour une eau de toilette masculine ou une lotion après rasage avec les ingrédients suivants :

| Ingrédients | Partie en poids |
|---|---|
| Undécalactone gamma | 200 |
| Essence de carotte | 300 |
| Essence de cumin | 300 |
| Estragole | 1200 |
| Essence de griffe de girofle | 1500 |
| Essence de mandarine | 3000 |
| Essence de sauge sclarée | 1800 |
| Prune synthétique | 1200 |
| | 9500 |

La base parfumante ainsi préparée peut être qualifiée de fruitée. Lorsqu'on ajoute à cette composition 500 parts de 12-pentyloxy-4,8-cyclododécadién-1-one, l'odeur de la composition devient plus fruitée, lactonique et épicée. De plus, le volume et la puissance de la composition sont nettement augmentés.
En remplaçant dans l'exemple ci-dessus l'ingrédient parfumant sus-mentionné par du 12-isobutoxy-4,8-cyclododécadién-1-one ou du 12-(2,2-diméthylpropoxy)-4,8-cyclododécadiène dans des proportions identiques, on a observé un effet analogue.

Exemple 5

On a préparé une composition parfumante de base pour détergent en poudre, avec les ingrédients suivants :

| Ingrédients | Partie en poids |
|---|---|
| Acétate de diméthylbenzylcarbinyle | 100 |
| Acétate de styrallyle | 100 |
| Aldéhyde anisique | 100 |
| Aldéhyde hexylcinnamique | 2500 |
| Citronellol | 1500 |
| Acétate de verdyle | 700 |
| Coumarine | 100 |
| Dihydromyrcenol | 200 |
| p-tert-Butylcyclohexylaldéhyde | 500 |
| IRALIA (marque enregistrée) [1] | 800 |
| LILIAL (marque enregistrée) [2] | 300 |
| MAYOL (marque enregistrée) [3] | 200 |
| Alcool phényléthylique | 1400 |
| TONALID (marque enregistrée) [4] | 500 |
| VERTOFIX COEUR (marque enregistrée) [5] | 500 |
| | 9500 |

1) alpha-méthylionone ; origine : Firmenich SA, Genève, Suisse
2) aldéhyde alpha-méthyl-para-tert-butyl hydrocinnamique ; origine : L. Givaudan SA, Vernier, Suisse
3) 4-isopropylcyclohexylméthanol ; origine : Firmenich SA, Genève, Suisse
4) 6-acétyl-1,1,3,4,4,6-hexaméthyl tétrahydronaphtalène ; origine : PFW
5) origine : IFF Inc.

Cette base parfumante peut être qualifiée de type floral-poudré-boisé-musqué. Lorsqu'on ajoute 500 parties en poids de l'un des corps parfumants nouveaux mentionnés dans l'exemple précédent, on observe que la composition prend plus de volume et un aspect aldéhydé fonctionnel, une note irisée se dégageant de la

composition.

**Revendications**

**1.** Utilisation à titre d'ingrédient parfumant d'un composé de formule

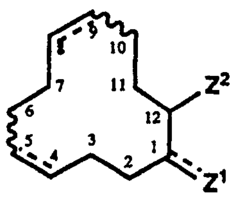

(I)

dans laquelle

a) les pointillés désignent l'emplacement d'une liaison simple ou double en positions 4 et 8 et d'une liaison carbone-oxygène simple en position 1, les lignes ondulées définissent une liaison C-C de configuration E ou Z lorsque la liaison en positions 4 et 8 est double, et les symboles $Z^1$ et $Z^2$, pris conjointement, définissent un groupe acétal ou cétal cyclique de formule

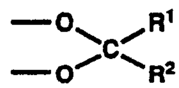

dans laquelle les symboles $R^1$ et $R^2$, identiques ou différents, représentent chacun un radical alkyle, de chaîne linéaire ou ramifiée, contenant de 1 à 6 atomes de carbone, ou l'un de ces symboles représente un atome d'hydrogène et l'autre représente un radical alkyle défini comme ci-dessus ;
ou dans laquelle

b) le symbole $Z^1$ représente un atome d'oxygène ou un groupe OH, les pointillés représentent l'emplacement d'une liaison simple ou double en positions 4 et 8, et d'une liaison carbone-oxygène simple ($Z^1$ = OH) ou double ($Z^1$ = O) en position 1, les lignes ondulées représentent une liaison C-C de configuration E ou Z lorsque la liaison en positions 4 et 8 est double, et le symbole $Z^2$ représente un groupe $OR^3$, où $R^3$ est un reste alkyle, de chaîne linéaire ou ramifiée, contenant de 1 à 6 atomes de carbone.

**2.** Composition parfumante contenant à titre d'ingrédient odoriférant un composé de formule (I) selon la revendication 1.

**3.** Produit parfumé contenant à titre d'ingrédient odoriférant un composé de formule (I) selon la revendication 1.

**4.** A titre de produit parfumé selon la revendication 8, un parfum ou une eau de toilette, un savon, un détergent, un produit d'entretien ou un produit cosmétique.

**5.** Procédé pour la préparation d'un composé de formule (I) selon la revendication 1, caractérisé en ce que :

a) on fait réagir, en présence d'un agent acide, un diol de formule

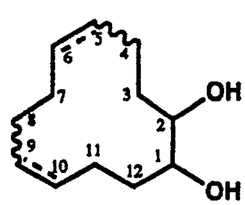

(II)

dans laquelle les pointillés désignent l'emplacement d'une liaison simple ou double en positions 5 et 9, et les lignes ondulées servent à définir une liaison C-C de configuration E ou Z lorsque la liaison en positions 5 et 9 est double, avec un composé de formule

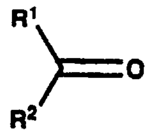

dans laquelle $R^1$ et $R^2$ sont définis comme à la revendication 1, pour obtenir un cétal de formule

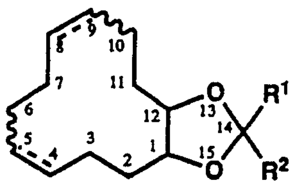

(III)

dans laquelle les lignes ondulées et les symboles $R^1$ et $R^2$ sont définis ci-dessus, et les pointillés désignent l'emplacement d'une liaison simple ou double en positions 4 et 8 ;

b) on fait réagir le cétal de formule (III) avec un agent réducteur pour obtenir un hydroxy-éther de formule

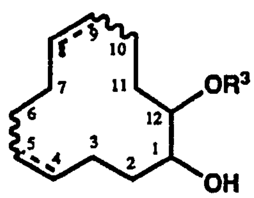

(IV)

dans laquelle les pointillés désignent l'emplacement d'une liaison simple ou double en positions 4 et 8, les lignes ondulées représentent une liaison C-C de configuration E ou Z lorsque la liaison en positions 4 et 8 est double, et le symbole $R^3$ est défini comme à la revendication 1 ;

c) on fait réagir l'hydroxy-éther de formule (IV) avec un agent oxydant pour obtenir un céto-éther de formule

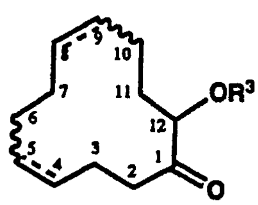

(V)

dans laquelle les pointillés, les lignes ondulées et le symbole $R^3$ sont définis comme en b).

**6.** Composé de formule (I) définie à la revendication 1, étant entendu que lorsque $Z^1$ est l'oxygène, $R^3$ ne peut pas être $CH_3$.

**7.** A titre d'un composé selon la revendication 6, un composé de formule (I) dans laquelle le symbole $Z^1$ est l'oxygène, les pointillés désignent l'emplacement d'une liaison double en positions 4 et 8, et d'une liaison carbone-oxygène double en position 1, les lignes ondulées définissent une liaison C-C de configuration E ou Z, et le symbole $Z^2$ représente un groupe $OR^3$, où $R^3$ est un radical alkyle, de chaîne linéaire ou ramifiée, contenant de 2 à 6 atomes de carbone.

**8.** A titre d'un composé selon la revendication 7, le 12-pentyloxy-4,8-cyclododécadién-1-one.

**9.** A titre d'un composé selon la revendication 7, le 12-(2,2-diméthylpropoxy)-4,8-cyclododécadién-1-one ou le 12-isobutoxy-4,8-cyclododécadién-1-one.

Office européen des brevets

# RAPPORT DE RECHERCHE EUROPEENNE

Numero de la demande

EP 89 12 0338

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 075 334 (L. GIVANDAN & CIE SOCIETE ANONYME)<br>* En entier *<br>--- | 1-5 | C 07 C 49/753<br>C 07 C 49/517<br>C 07 C 43/196<br>C 07 D 317/44<br>C 11 B 9/00<br>A 61 K 7/46<br>C 07 C 45/29<br>C 07 C 45/30<br>C 07 C 41/28 |
| X | FR-A-1 264 032 (SOCIETE DES USINES CHIMIQUES RHONE-POULENC)<br>* En entier *<br>--- | 1,6 | |
| X | GB-A-2 000 127 (COALITE AND CHEMICAL PRODUCTS LTD)<br>* En entier *<br>--- | 1 | |
| X | CHEMICAL ABSTRACTS, vol. 106, 1987, page 592, résumé no. 49666t, Columbus, Ohio, US; & JP-A-61 134 336 (SANYO-KOKUSAKU PULP CO. LTD) 21-06-1986<br>----- | 1 | |
| | | | **DOMAINES TECHNIQUES RECHERCHES (Int. Cl.5)** |
| | | | C 07 C 49/00<br>C 07 C 43/00<br>C 07 C 45/00<br>C 07 C 41/00<br>C 07 D 317/00 |

Le présent rapport a été établi pour toutes les revendications

| Lieu de la recherche | Date d'achèvement de la recherche | Examinateur |
|---|---|---|
| LA HAYE | 31-01-1990 | BONNEVALLE E.I.H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)